# EUROPÄISCHE PATENTSCHRIFT

(11) **EP 1 337 498 B1**
(45) Veröffentlichungstag und Bekanntmachung des Hinweises auf die Patenterteilung: **14.11.2007**
(21) Anmeldenummer: 01983561.0
(22) Anmeldetag: 19.10.2001
(51) Int. Cl.: C04B 40/00, F25C 3/00, B28C 5/46

(54) **VERFAHREN UND VORRICHTUNG ZUR BETONHERSTELLUNG**
METHOD AND DEVICE FOR THE PRODUCTION OF CONCRETE
PROCEDE ET DISPOSITIF DE PREPARATION DU BETON

(30) Priorität: 03.11.2000 DE 10054563
(43) Veröffentlichungstag der Anmeldung: 27.08.2003
(73) Patentinhaber: Air Liquide Deutschland GmbH, 40235 Düsseldorf (DE); Max Boegl Bauunternehmung GmbH & Co. KG, 92301 Neumarkt (DE); Messer Group GmbH, 65843 Sulzbach (DE)
(72) Erfinder: LINDNER, Erich, 91275 Aucherbach (DE); REICHEL, Dieter, 92318 Neumarkt (DE); TAUCHMANN, Jens, 13189 Berlin (DE); WEICHMANN, Thomas, 92367 Pilsach (DE)
(74) Vertreter: Kahlhöfer, Hermann
(86) Internationale Anmeldenummer: PCT/EP2001/012100
(87) Internationale Veröffentlichungsnummer: WO 2002/036523

(56) Entgegenhaltungen:
- WO-A-98/03828
- DE-A- 2 013 819
- DE-A- 2 342 658
- FR-A- 2 195 184
- US-A- 4 830 669
- DATABASE WPI Week 198925 Derwent Publications Ltd., London, GB; AN 1989-183935 XP002186091 & JP 01 123705 A (WATARI SHOKAI)
- PATENT ABSTRACTS OF JAPAN vol. 015, no. 303 (M-1142), 2. August 1991 (1991-08-02) & JP 03 110364 A (YAMAGUCHI SAKUTARO), 10. Mai 1991 (1991-05-10)
- DATABASE WPI Week 199006 Derwent Publications Ltd., London, GB; AN 1990-040555 XP002186092 & JP 01 317143 A (SHIMIZU CONSTRUCTION)

## Beschreibung

Die Erfindung betrifft ein Verfahren sowie eine Vorrichtung zur Betonherstellung.

Um die Bildung von Rissen und Spannungen beim Bau von großen Betonkörpern wie Brückenwiderlagern, Staumauern, Pylonen u. dergl. zu vermeiden, ist es üblich, für diese Bauwerke gekühlten Frischbeton einzusetzen. Hierdurch kann die bei der Hydration des Zements freiwerdende Wärme besser kompensiert und gleichmäßiger abgeführt und so Spannungsdifferenzen im Beton vermieden werden. Außerdem ist die Verarbeitung von ungekühltem Frischbeton bei Außentemperaturen über 25°C problematisch, da die bei derart hohen Außentemperaturen resultierende Peaktemperatur des Betons beim Abbinden oberhalb von 60°C liegen und somit zu Festigkeitsverlusten führen kann. Durch die Kühlung wird das Überschreiten diese Grenze vermieden.

Zur Herstellung von gekühltem Frischbeton ist es bekannt, den bereits fertig angemachten Frischbeton durch Zugabe von Scherbeneis oder flüssigen Stickstoff im stationären oder mobilen Mischer oder in der rotierenden Trommel eines Betontransportfahrzeugs auf die gewünschte Temperatur zu kühlen. Einen zusammenfassenden Überblick über bekannte Kühlverfahren geben die Artikel "Kühlen von Frischbeton mit flüssigem Stickstoff' von W. Trappmann und W. Duesberg in "gas aktuell", 25 (1983), S. 15 und "Frischbeton mit flüssigem Stickstoff' von D. Rebhan in "Betonwerk + Fertigteil-Technik", Heft 8/1981, S. 507. Ein entsprechendes Verfahren ist auch aus der WO-A-98/03828 bekannt.

Bei der Verwendung von Eis als Kühlmittel besteht die Gefahr von Inhomogenitätserscheinungen infolge von Wassereinschlüssen im Beton. Auch ist der Kühleffekt äußerst begrenzt, da die Kühlung von Scherbeneis auf Temperaturen T << 0°C nur mit hohem und in den meisten Fällen wirtschaftlich nicht zu rechtfertigendem Aufwand verbunden ist. Weiterhin ist das Scherbeneis nicht gut löslich, und es kommt zu einer Verlängerung der Mischzeiten, was insbesondere in den Mischwerken zu erheblichen Verringerung der Produktionsleistung führt.

Nachteilig an der Kühlung mittels tiefkaltem verflüssigtem Stickstoff (LN₂) ist, dass dieses Verfahren nur einen geringen Wirkungsgrad besitzt, da verhältnismäßig große Mengen LN₂ innerhalb kurzer Zeit in die jeweilige Charge eingetragen werden müssen, um den gewünschten Abkühleffekt zu erreichen. Der schlechte Wirkungsgrad bei dieser Kühlmethode liegt darin begründet, dass primär nur die Verdampfungswärme des flüssigen Stickstoffs genutzt werden kann und die für den Wärmeaustausch zur Verfügung stehende Oberfläche begrenzt ist. Durch das schnelle Verdampfen des Stickstoffs werden ferner innerhalb kürzester Zeit große Gasvolumina freigesetzt, was zu lokalen Verpuffungen und Herausschleudern aus dem Mischer führen kann. Darüber hinaus kann der Frischbeton infolge lokaler kritischer Unterkühlung Frostschäden nehmen.

In der JP 61201681 A wird vorgeschlagen, die Ausgangsstoffe vor der Zugabe von Zement und Wasser durch Zugabe eines verflüssigten Gases, wie Stickstoff oder Kohlendioxid, abzukühlen. Dieses Verfahren ist allerdings nur begrenzt einsetzbar, da das gekühlte Medium wärme- und feuchtigkeitsisoliert gespeichert werden muss. Eine Kühlung von Stoffen mit hohem Feuchtigkeitsgehalt auf Temperaturen unterhalb von 0°C ist im übrigen nicht möglich.

Aufgabe der vorliegenden Erfindung ist daher, ein Verfahren sowie eine Vorrichtung zur Herstellung von Beton anzugeben, das die Nachteile der vorbekannten Kühlverfahren vermeidet und insbesondere einen wirtschaftlichen Einsatz des verwendeten Kühlmittels bei gleichzeitiger hoher Kühlleistung erlaubt.

Gelöst ist diese Aufgabe zum einen durch ein Verfahren mit den Merkmalen des Patentanspruchs 1, zum anderen durch eine Vorrichtung mit den Merkmalen des Anspruchs 7.

Beim erfindungsgemäßen Verfahren wird also ein Teil oder die gesamte Menge des dem aus Bindemittel, etwa Zement, und Zuschlagsstoffen, wie Kies, Sand, Flugasche u. dergl. bestehenden Bindemittelgemisches zugesetzten Wasser in Form eines zuvor erzeugten, aus Schneekristallen bestehenden Kälteträgers zugeführt.

Als "Schneekristalle" sollen hier und im folgenden Partikel aus gefrorenem Wasser verstanden werden, die in einer kalten Atmosphäre erzeugt werden.

Derartige Schneekristalle weisen gegenüber Scherbeneis den Vorteil einer größeren Oberfläche bei einem gleichzeitig geringerem spezifischem Gewicht auf. Die Übertragung der beim Abbinden erzeugten Prozesswärme erfolgt dadurch sehr viel rascher als beim Scherbeneis. Des weiteren werden Inhomogenitätserscheinungen durch Wassereinschlüsse weitgehend vermieden.

Bei dem erfindungsgemäßen Verfahren zum Herstellen von Schnee-/Eiskristallen wird aus Wasser, einem Treibgas sowie einem Kältemittel ein Kaltgas erzeugt, das in einer Sprühkammer in Form eines Kaltgasstrahls versprüht wird. Dieser Kaltgasstrahl weist eine Temperatur auf, die deutlich unterhalb der Gefriertemperatur des Wassers liegt. Das im Kaltgasstrahl vorliegende Wasser gefriert zu Kristallen, die in der Folge einem Bindemittelgemisch zur Herstellung von Frischbeton beigemengt werden. Größe, Temperatur und Oberflächenbeschaffenheit der Kristalle werden maßgeblich von der Zusammensetzung des Kaltgases und der Temperatur des Kaltgasstrahls bestimmt. Problemlos ist auch die Herstellung von Kristallen mit Temperaturen von weit unter 0°C möglich. Insbesondere ergeben sich bei Temperaturen von weniger als minus 30°C besonders gute Transporteigenschaften der erzeugten Kristalle, da bei diesen Temperaturen keine mikroskopischen Domänen flüssigen Wassers auf der Oberfläche der Schneekristalle mehr vorhanden sind, durch die ansonsten ein Zusammenkleben der Schneekristalle begünstigt werden würden. Die Kältewirkung des eingesetzten Kältemittels wird somit in einem weit höheren Maße ausgenutzt, als bei konventionellen Kühlverfahren.

Vorteilhafterweise wird der Kaltgasstrahl in Rotation versetzt. Hierdurch wird der Strahlweg in der Strahlkammer verlängert und dadurch eine größere Homogenität der erzeugten Kristalle erreicht.

Um die Zieltemperatur des Frischbetons schnell zu erreichen, wird der in der in der Sprühkammer erzeugte Kälteträger einer Nachkühlung unterzogen. Bei Wahl eines geeigneten Kühlmittels, etwa flüssiger Stickstoff, lassen sich so Schneekristalltemperaturen von bis zu -190°C erzeugen. Als bevorzugtes Kühlmittel, sowohl für die Herstellung des Kaltgases wie auch für die Nachkühlung, kommt ein tiefkaltes, verflüssigtes Gas zum Einsatz. Unter Umwelt- und Wirtschaftlichkeitsaspekten empfehlen sich hierbei insbesondere flüssiger Stickstoff oder flüssiges Kohlendioxid.

Als Treibgas zur Herstellung des Kaltgases bzw. des Kaltgasstrahls kommt vorteilhafterweise gasförmiger Stickstoff zum Einsatz. Durch die Verwendung des - selbst nur schwer wasserlöslichen - Stickstoffs wird verhindert, dass sich Sauerstoff in Wasser löst.

Das eingesetzte flüssige Kühlmedium dient vorzugsweise gleichzeitig dem Transport des Kälteträgers aus der Sprühkammer an das Bindemittelgemisch. Hierdurch kann ein Austrag des Kälteträgers weitestgehend ohne Verdichtung, Verklumpung und Veränderung der Kristallstruktur der Schnee-/Eiskristalle erreicht werden.

Die erfindungsgemäße Vorrichtung gemäß Anspruch 8 umfasst eine Mischeinrichtung, in der Wasser, ein Treibgas, beispielsweise Stickstoff, und ein Kühlmittel, beispielsweise verflüssigter Stickstoff, zu einem Kaltgas gemischt wird, und die mit einer in einer Sprühkammer aufgenommenen Sprühvorrichtung in Strömungsverbindung steht. In der Sprühkammer entstehen beim Versprühen des Kaltgases aus dessen Wasserbestandteil Schnee-/Eiskristalle. Durch das Einsprühen des Kaltgases in die Sprühkammer entsteht in dieser zugleich eine inerte und unterkühlte Atmosphäre, die die Entstehung von Schneekristallen mit großer Oberfläche und geringem spezifischem Gewicht begünstigt. Der in der Sprühkammer erzeugte Kälteträger wird einer Mischkammer zugeführt, in der er mit einem Bindemittelgemisch zu Frischbeton vermengbar ist.

Zweckmäßigerweise ist der Sprühkammer eine Kühleinrichtung zugeordnet, mittels der der in der Sprühkammer erzeugte Schnee auf einen vorbestimmten Temperaturwert weiter abgekühlt werden kann. Durch die Wahl eines geeigneten Kälteträgers in der Kühleinrichtung können Temperaturen von minus 30°C bis zu minus 190°C erreicht werden.

Eine vorteilhafte Weiterbildung der Erfindung sieht vor, die Mischeinrichtung zur Erzeugung des Kaltgases und/oder die Mischkammer zur Erzeugung des Frischbetons mit einer Steuereinrichtung zu verbinden, mittels der die Zusammensetzung des Kaltgases und/oder des Frischbetons nach einem vorbestimmten Programm einstellbar ist. Die Zusammensetzung des Kaltgases bestimmt- neben der Düsengeometrie der Sprühdüse - maßgeblich die Konsistenz und die Temperatur des erzeugten Kälteträgers. Die Steuerung der Zufuhr erfolgt dabei durch geeignete, von der Steuereinrichtung angesteuerte Ventile an der Mischeinrichtung bzw. der Mischkammer.

Anhand der Zeichnung soll nachfolgend ein Ausführungsbeispiel der Erfindung näher erläutert werden.

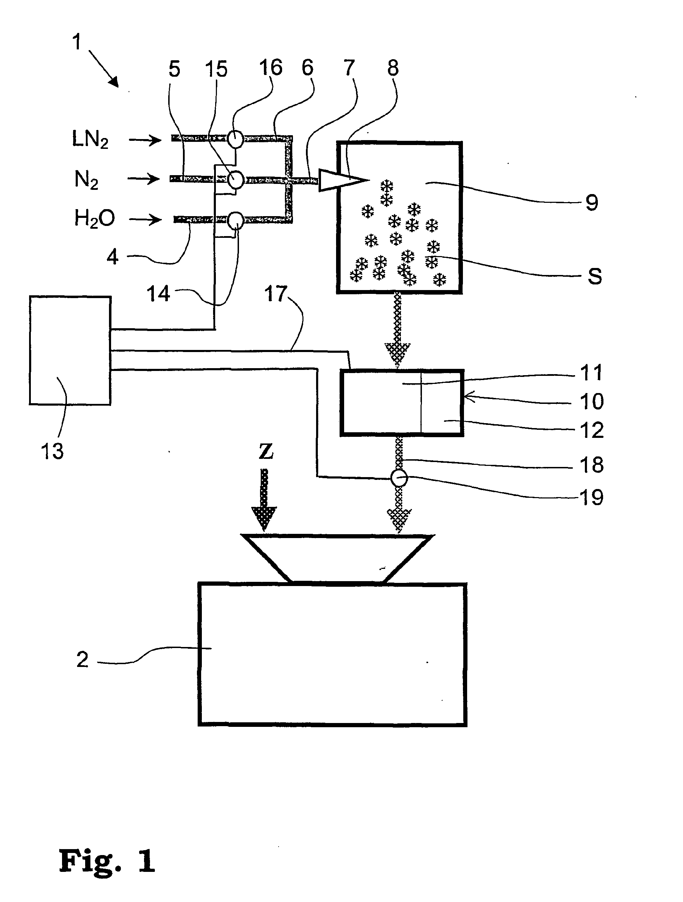

Die einzige Zeichnung (Fig. 1) zeigt schematisch die Wirkungsweise einer erfindungsgemäßen Vorrichtung zum Erzeugen von Frischbeton.

Die Vorrichtung 1 weist eine an sich bekannte Mischkammer 2 auf, in der die zur Herstellung von Frischbeton erforderlichen Zuschlagstoffe, wie Sand, Kies, Flugasche, sowie Zement eingebracht und zu einem Bindemittelgemisch Z vermengt werden. Bei der Mischkammer kann es sich beispielsweise um eine mobile oder stationäre Mischanlage handeln. Anstelle des bei üblichen Herstellungsverfahren eingesetzten flüssigen Wassers wird dem Bindemittelgemisch in der Mischkammer 2 ein unterkühlter Kälteträger S zugegeben, dessen Herstellung im folgenden beschrieben wird.

Zur Herstellung des Kälteträgers S werden Wasser sowie gasförmiger und flüssiger Stickstoff über entsprechende Leitungen 4,5,6 herangeführt, in einer Mischstrecke 7 zu einem Kaltgasgemisch durchmischt und einer Sprühdüse 8 zugeführt, die in einer Sprühkammer 9 angeordnet ist. Anstelle einer mehr oder weniger langen Mischstrecke können die Leitungen 4,5,6 auch unmittelbar in die Sprühdüse 8 münden, die zu diesem Zweck als Dreistoffdüse ausgebildet ist. Die Erfindung ist im übrigen nicht auf flüssigem Stickstoff als Kältemittel für die Herstellung des Kaltgasgemisches beschränkt, vielmehr können hierzu auch andere bekannte Kältemittel, insbesondere andere verflüssigte Gase herangezogen werden. Auch kann anstelle einer Dreistoffdüse ein anderes Mehrdüsensystem verwendet werden.

Mittels der Sprühdüse 8 wird das Kaltgasgemisch in Form eines in das Innere der Sprühkammer 9 gerichteten Kaltgasstrahls ausgebracht, wobei der Kaltgasstrahl in eine Rotation um die eigene Achse versetzt wird, um den Strahlweg zu verlängern. Durch den Eintrag des Kaltgasstrahl bildet sich im Innern der Sprühkammer 9 bereits nach kurzer Zeit eine unterkühlte Atmosphäre aus. Das im Kaltgasstrahl enthaltene Wasser gefriert und geht im Innern der Sprühkammer 9 ain Form von Schneekristallen nieder, dem Kälteträger S. Die kalte, inerte Atmosphäre im Innern der Sprühkammer 9 begünstigt dabei das Ausbilden von Kristallen mit großer Oberfläche und kleinem spezifischen Gewicht. Größe, Konsistenz und Temperatur der Kristalle werden dabei insbesondere durch das Mischungsverhältnis aus gasförmigem und flüssigem Stickstoff sowie Wasser im Kaltgasgemisch bestimmt.

Die in der Sprühkammer 9 gebildeten Schneekristalle werden im Ausführungsbeispiel einer Nachkühleinrichtung 10 zugeführt, in der der Kälteträger S weiter abgekühlt wird. Die Nachkühleinrichtung 10 besteht aus einer Kühlkammer 11 für das zu kühlende Gut, die mit einem Kälteträger 12 in thermischen Kontakt steht. Es ist im Rahmen der Erfindung auch möglich, die Sprühkammer 9 mittels des darin eingetragenen flüssigen Stickstoffs unmittelbar als Kühlkammer 11 der Nachkühleinrichtung 10 einzusetzen und/oder Flüssigstickstoff aus dem Kaltgasstrahl auch als Transportvehikel für die Schneekristalle zu verwenden. Wird die Nachkühleinrichtung 10 mit flüssigem Stickstoff als Kälteträger 12 betrieben, können Temperaturen bis hinab zu -190°C erzielt werden. Bei Temperaturen unter -30°C, beispielsweise minus 40°C, lassen sich die Schneekristalle besonders gut transportieren. Der so gekühlte Kälteträger S wird der Mischkammer 2 zugeführt und dort in bekannter Weise mit Zugabestoffen und mit Zement zu Frischbeton gemischt.

Die große Oberfläche der Schneekristalle des Kälteträgers S ermöglicht eine effektive und rasche Aufnahme der beim Abbindevorgang des Zements entstehenden Prozesswärme. Durch Variation von Temperatur und Menge des eingesetzten Kälteträgers S können Frischbetontemperaturen von bis hinab zu 0°C erzielt werden.

Eine elektronische Steuerung 13 ermöglicht die Herstellung des Kälteträgers S bzw. Frischbeton nach einem vorgegebenen Programm. Die elektronische Steuerung 13 ist mit ansteuerbaren Ventilen 14,15,16, beispielsweise Magnetventilen, in den Leitungen 4,5,6 verbunden, mittels derer das Mischungsverhältnis und/oder der jeweilige Druck in den Leitungen 4,5,6 eingestellt werden kann. Eine Steuerleitung 17 dient zur Temperaturregelung in der Nachkühleinrichtung 10. Die Zuführung 18 des Kälteträgers S zur Mischkammer 2 ist gleichfalls mit einem Magnetventil 19 ausgerüstet, das von der Steuerung 13 angesteuert werden kann. Auf diese Weise kann die Temperatur, Konsistenz und Menge des dem Bindemittelgemisch Z zugeführten Kälteträgers S genau und zuverlässig eingestellt und beispielsweise so gewählt werden, dass der erzeugte Frischbeton einen gewissen Temperaturwert, beispielsweise 0°C aufweist. Um den vorgegebenen Temperaturwert während der Dauer der Herstellung einer Betonmenge einzuhalten, wird die mittels eines geeigneten Messgeräts kontinuierlich oder regelmäßig gemessene Temperatur des Frischbetons als Stellgröße eingeführt, auf die durch die Einstellung der Temperatur des zugeführten Kälteträgers S hin laufend geregelt wird.

Das erfindungsgemäße Verfahren ermöglicht die Umwandlung des Zugabewassers in Kälteträger S innerhalb weniger Minuten. Infolge der großen Oberfläche der Schneekristalle erfolgt die Kälteübertragung beim Nachkühlen sehr effektiv, so dass auch dieser Vorgang nur wenige Minuten in Anspruch nimmt.

### Bezugszeichenliste

- 1.: Vorrichtung
- 2.: Mischkammer
- 3.: -
- 4.: Leitung
- 5.: Leitung
- 6.: Leitung
- 7.: Mischstrecke
- 8.: Sprühdüse
- 9.: Sprühkammer
- 10.: Nachkühleinrichtung
- 11.: Kühlkammer
- 12.: Kälteträger
- 13.: elektronische Steuerung
- 14.: Ventil
- 15.: Ventil
- 16.: Ventil
- 17.: Steuerleitung
- 18.: Zuführleitung
- 19.: Magnetventil
- S: Kälteträger
- Z: Bindemittelgemisch

## Patentansprüche

1. Verfahren zur Betonherstellung, bei dem ein Bindemittel, etwa Zement, mit Zuschlagstoffen zu einem Bindemittelgemisch (Z) vermengt und durch Zugabe von Wasser an das Bindemittelgemisch (Z) Frischbeton hergestellt wird,
wobei das Wasser dem Bindemittelgemisch (Z) zumindest zum Teil in Form µeines aus Schneekristallen bestehenden Kälteträgers (S) zugeführt wird, **dadurch gekennzeichnet, dass** zur Herstellung des Kälteträgers (S)
- Wasser mit einem Treibgas und einem Kühlmittel zu einem Kaltgasgemisch vermischt wird,
- das Kaltgasgemisch als Kaltgasstrahl in eine Sprühkammer (9) eingesprüht wird, wobei das Wasser zu Schneekristallen gefriert.

2. Verfahren nach Anspruch 1, **dadurch gekennzeichnet, dass** der Kaltgasstrahl beim Einsprühen in die Sprühkammer (9) in Rotation versetzt wird.

3. Verfahren nach Anspruch 1 oder 2, **dadurch gekennzeichnet, dass** der Kälteträger (S) vor der Zuführung an das Bindemittelgemisch (Z) in einem Verfahren zur Nachkühlung auf eine vorgegebene Temperatur von unterhalb von minus 30°C gebracht wird.

4. Verfahren nach einem der Ansprüche 1. bis 3, **dadurch gekennzeichnet, dass** als Kühlmittel für das Kaltgasgemisch und/oder für die Nachkühlung des Kälteträgers (S) ein tiefkaltes, verflüssigtes Gas, etwa verflüssigter Stickstoff oder verflüssigtes Kohlendioxid eingesetzt wird.

5. Verfahren nach einem der vorhergehenden Ansprüche, dass als Treibgas für das Kaltgasgemisch Stickstoff und/oder Luft eingesetzt wird.

6. Verfahren nach einem der Ansprüche 1 bis 5, **dadurch gekennzeichnet, dass** das Kühlmittel zum Transport des erzeugten Kälteträgers (S) an das Bindemittelgemisch (Z) eingesetzt wird.

7. Vorrichtung zur Betonherstellung,
- mit einer Mischeinrichtung (7) zum Vermischen von Wasser, einem Treibgas und einem Kühlmittel zu einem Kaltgasgemisch,
- mit einem mit der Mischeinrichtung (7) strömungsverbundenen und in einer Sprühkammer (9) aufgenommenen Sprühdüse (8), mittels der das Kaltgasgemisch unter Bildung eines zumindest im wesentlichen aus Schneekristallen bestehenden Kälteträgers (S) versprühbar ist und
- mit einer Mischkammer (2), in der in der Sprühkammer (9) erzeugter Kälteträger (S) mit einem Bindemittelgemisch (Z) zu Frischbeton vermengbar ist, **dadurch gekennzeichnet, dass** der Sprühkammer (9) eine Kühleinrichtung (11) zum Nachkühlen des in der Sprühkammer (9) erzeugten Kälteträger (S) zugeordnet ist.

8. Vorrichtung nach Anspruch 7, **gekennzeichnet durch** eine mit der Mischeinrichtung (7) und/oder der Mischkammer (2) in Wirkverbindung stehende Steuereinrichtung (12), mittels der die Temperatur und/oder die Zusammensetzung des Kaltgasgemisches und/oder des Frischbetons einstellbar ist.

## Claims

1. Method for concrete production, in which a binder, for example cement, is intermingled with additives to form a binder mixture (Z) and fresh concrete is produced by the addition of water to the binder mixture (Z), the water being supplied to the binder mixture (Z) at least partially in the form of a refrigerant (S) consisting of snow crystals, **characterized in that**, to produce the refrigerant (S),
- water is intermixed with a propellant gas and with a coolant to form a cold-gas mixture,
- the cold-gas mixture is injected as a cold-gas jet into a spray chamber (9), the water freezing into snow crystals.

2. Method according to Claim 1, **characterized in that** the cold-gas jet is set in rotation when it is being injected into the spray chamber (9).

3. Method according to Claim 1 or 2, **characterized in that** the refrigerant (S), before being supplied to the binder mixture (Z), is brought to a predetermined temperature of below minus 30°C in an aftercooling method.

4. Method according to one of Claims 1 to 3, **characterized in that** a low-temperature liquefied gas, for example liquefied nitrogen or liquefied carbon dioxide, is used as a coolant for the cold-gas mixture and/or for the aftercooling of the refrigerant (S).

5. Method according to one of the preceding claims, **characterized in that** nitrogen and/or air is used as a propellant gas for the cold-gas mixture.

6. Method according to one of Claims 1 to 5, **characterized in that** the coolant is used for transporting the generated refrigerant (S) to the binder mixture (Z).

7. Apparatus for concrete production,
- with a mixing device (7) for the intermixing of water, a propellant gas and a coolant to form a cold-gas mixture,
- with a spray nozzle (8) which is flow-connected to the mixing device (7) and is received in a spray chamber (9) and by means of which the cold-gas mixture can be sprayed so as to form a refrigerant (S) consisting at least essentially of snow crystals, and
- with a mixing chamber (2), in which refrigerant (S) generated in the spray chamber (9) can be intermingled with a binder mixture (Z) to form fresh concrete, **characterized in that** the spray chamber (9) is assigned a cooling device (11) for the aftercooling of the refrigerant (S) generated in the spray chamber (9).

8. Apparatus according to Claim 7, **characterized by** a control device (12) which is operatively connected to the mixing device (7) and/or the mixing chamber (2) and by means of which the temperature and/or the composition of the cold-gas mixture and/or of the fresh concrete can be set.

## Revendications

1. Procédé de préparation de béton, dans lequel un liant, tel que du ciment, est mélangé à des granulats pour former un mélange liant (Z) et du béton frais est préparé par ajout d'eau au mélange liant (Z), l'eau étant introduite dans le mélange liant (Z) au moins en partie sous forme d'un caloporteur frigorigène (S) constitué de cristaux de neige, **caractérisé en ce que**, pour la préparation du caloporteur frigorigène (S),
- l'eau est mélangée à un gaz propulseur et un agent réfrigérant pour former un mélange de gaz froid,
- le mélange de gaz froid est pulvérisé dans une chambre de pulvérisation (9) sous forme de jet de gaz froid, l'eau gelant pour former des cristaux de neige.

2. Procédé selon la revendication 1, **caractérisé en ce que** le jet de gaz froid est mis en rotation lors de la pulvérisation dans la chambre de pulvérisation (9).

3. Procédé selon la revendication 1 ou 2, **caractérisé en ce que** le caloporteur frigorigène (S), avant d'être introduit dans le mélange liant (Z), est amené à une température prédéfinie de moins de moins 30 °C dans un procédé de post-refroidissement.

4. Procédé selon l'une quelconque des revendications 1 à 3, **caractérisé en ce qu'**un gaz liquéfié à basse température, tel que de l'azote liquéfié, ou du dioxyde de carbone liquéfié, est utilisé comme agent réfrigérant pour le mélange de gaz froid et/ou pour le post-refroidissement du caloporteur frigorigène (S).

5. Procédé selon l'une quelconque des revendications précédentes, **caractérisé en ce que** de l'azote et/ou de l'air est utilisé comme gaz propulseur pour le mélange de gaz froid.

6. Procédé selon l'une quelconque des revendications 1 à 5, **caractérisé en ce que** l'agent réfrigérant est utilisé pour le transport du caloporteur frigorigène (S) produit jusqu'au mélange liant (Z).

7. Dispositif de préparation de béton, comprenant :
- un dispositif mélangeur (7) pour mélanger de l'eau, un gaz propulseur et un agent réfrigérant pour former un mélange de gaz froid,
- une buse de pulvérisation (8) logée dans une chambre de pulvérisation (9) et en communication fluidique avec le dispositif mélangeur (7) au moyen de laquelle le mélange de gaz froid peut être pulvérisé en formant un caloporteur frigorigène (S) constitué au moins essentiellement de cristaux de neige, et
- une chambre de mélange (2) dans laquelle le caloporteur frigorigène (S) produit dans la chambre de pulvérisation (9) peut être mélangé avec un mélange liant (Z) pour former du béton frais, **caractérisé en ce qu'**un dispositif réfrigérant (11) destiné à refroidir ultérieurement le caloporteur frigorigène (S) produit dans la chambre de pulvérisation (9) est associé à la chambre de pulvérisation (9).

8. Dispositif selon la revendication 7, **caractérisé par** un dispositif de commande (12) en communication active avec le dispositif mélangeur (7) et/ou la chambre de mélange (2), au moyen duquel la température et/ou la composition du mélange de gaz froid et/ou du béton frais peuvent être ajustées.
